# EUROPEAN PATENT SPECIFICATION

(11) **EP 3 940 035 B1**
(45) Date of publication and mention of the grant of the patent: **18.10.2023**
(21) Application number: 20185439.5
(22) Date of filing: 13.07.2020
(51) Int. Cl.: C09J 9/02, H01B 1/22, A61L 24/00, C08F 265/06, C09J 4/06, C09J 151/00

(54) **ELECTRICALLY CONDUCTIVE ADHESIVE**
ELEKTRISCH LEITFÄHIGER KLEBSTOFF
ADHÉSIF ÉLECTROCONDUCTEUR

(43) Date of publication of application: 19.01.2022
(73) Proprietor: Heraeus Deutschland GmbH & Co. KG, 63450 Hanau (DE)
(72) Inventor: SCHIBLI, Stefan, 63450 Hanau (DE); NEUMANN, Christian, 63450 Hanau (DE); VOGT, Sebastian, 61273 Wehrheim (DE); REISINGER, Andreas, 63450 Hanau (DE)
(74) Representative: Maiwald GmbH

(56) References cited:
- EP-A1- 2 990 061
- WO-A1-2019/206417
- CN-A- 110 218 524
- US-A- 4 127 699

## Description

### FIELD OF THE INVENTION

The invention relates to an electrically conductive adhesive, a kit for an electrically conductive adhesive, an implantable device comprising the electrically conductive adhesive or a cured form thereof, and the use of an electrically conductive adhesive for connecting electronic parts in an implantable medical device.

### BACKGROUND OF THE INVENTION

Active implantable medical devices contain electrical contact elements which connect the electronics of the implant to wires, electrodes or electrode arrays. The wires or electrodes are in contact with nerves or tissues of an organ or a specific body part of the patient carrying the implant. An electrical impulse or electrical current can be passed from the electronics of the implant to the nerves or tissues of the patient for microstimulating this specific part of the body.

The connections between the electronics of the implant and the wires or electrodes have to be durable and reliable in function as any failure may lead to severe consequences for the health of the patient. Furthermore, the materials used for connecting the parts of the implant have to be safe from a medical perspective for a long-term use in the body of the patient.

The connection of the electronics of the implant with wires or electrodes is usually further complicated by the fact that the electronics of the implant have to be secured in a hermetically sealed housing to avoid contact with body fluids. The electronics of the implant are then connected to the wires or electrodes via a feedthrough which electrically connects the interior of the housing to the outside. The electrical connection of wires or electrodes to a feedthrough, especially to a ceramic/metal composite (cermet) feedthrough, is challenging.

In the state of the art, electrical connections in active medical implants are often created by welding or brazing processes. As such processes require the use of high temperature, the choice of the materials of the implant can be limited. Furthermore, use of high temperature for assembling the implantable device can lead to fractures or microfractures in the materials of the device, which may ultimately limit its lifetime. Another possibility to create electrical connections in implantable devices is by mechanically fasten, e.g. crimping, electric pieces to one another.

As the trend in active implantable medical devices is towards miniaturization, mechanically connecting electrical components is very difficult, and will become even more so in the near future. Further to this, a mechanical connection has a risk of fatigue and therefore creates a risk of failure, which should be avoided.

In view of the above, there is a continuing need in the art for new electrical connections, which may be used in implantable medical devices, especially active implantable medical devices. In particular, the electrical connections should be biocompatible to allow for a long-term use in the body of a patient, the electrical connection should be suitable for miniaturization, and should be applicable under comparatively mild conditions.

Therefore, the present invention is directed to the provision of a new electrical connection, which may be used in implantable medical devices.

WO 2019/206417 describes an electrically conductive adhesive for attaching solar cells. Said adhesive comprises (a) a resin selected from epoxy (meth)acrylate, (poly)ester (meth)acrylate, urethane (meth)acrylate, silicone (meth)acrylate, poly(iso)butylene (meth)acrylate, (poly)isoprene (meth)acrylate, polybutylene (meth)acrylate and mixtures thereof; b) a diluting acrylic monomer, preferably a (meth)acrylate monomer comprising two or more (meth)acrylate groups; c) an electrically conductive filler and d) a curing agent. EP 2 990 061 describes a radiopaque composition for use in the medical field, e.g. as a bone cement, said composition comprising methyl methacrylate as monomer, radiopaque filler particles comprising gold dispersed in a polymethyl methacrylate polymer matrix, and a curing system for curing the monomer, said curing system preferably comprising an initiator and an accelerator.

### SUMMARY OF THE INVENTION

The invention provides an electrically conductive adhesive comprising
a) a (meth)acrylate monomer,
b) a polymer being soluble in the (meth)acrylate monomer, and wherein the polymer is selected from the group consisting of poly((meth)acrylates), poly((meth)acrylate) copolymers, and a mixture thereof,
c) a biocompatible metal having a median particle size d₅₀ of below 50 µm, and
d) a polymerization initiator.

The inventors found that a composition comprising a (meth)acrylate monomer, a polymer being soluble in the (meth)acrylate monomer and selected from the group consisting of poly((meth)acrylates), poly((meth)acrylate) copolymers, and a mixture thereof, a biocompatible metal having a median particle size d₅₀ of below 50 µm, and a polymerization initiator, provides an electrically conductive adhesive. The inventive adhesive can be useful for connecting electrical parts e.g. in an implantable medical device.

Furthermore, it has been found that an electrically conductive adhesive can successfully be based on a biocompatible metal and a (meth)acrylate monomer as a polymerizable pre-cursor of the adhesive. (Meth)acrylate monomers, and their polymers or copolymers, are used in the art for preparing bone cements, which may be applied e.g. for fixing endoprostheses in the human skeleton. Therefore, (meth)acrylate monomers, and especially their polymers or copolymers, also show biocompatibility. In view thereof, the inventive adhesive successfully combines the function of a conductive adhesive with a suitability for use in implantable medical device.

In another aspect of the invention, a kit for preparing an electrically conductive adhesive is provided. The kit comprises a component A and a component B,
wherein component A comprises a (meth)acrylate monomer, and
wherein component B comprises a polymer being soluble in the (meth)acrylate monomer of component A, and wherein the polymer is selected from the group consisting of poly((meth)acrylates), poly((meth)acrylate) copolymers, and a mixture thereof, a biocompatible metal having a median particle size d₅₀of below 50 µm, and a polymerization initiator.

In yet another aspect of the present invention, an implantable medical device is provided. The implantable medical device comprises at least two electronic parts,
wherein the at least two electronic parts are connected by
a) the electrically conductive adhesive according to any one of claims 1 to 12, or a cured form thereof, or
b) the electrically conductive adhesive prepared from the kit according to claim 13, or a cured form thereof.

A further aspect of the present invention refers to the use of an electrically conductive adhesive according to the present invention, or a kit according to the present invention, for connecting at least two electronic parts in an implantable medical device.

It should be understood that for the purposes of the present invention, the following terms have the following meanings:
The term "(meth)acrylate" is meant to encompass the options of "methacrylate" and "acrylate". "Methacrylate" relates to a methacrylic acid ester and "acrylate" to an acrylic acid ester.

The term "biocompatible" in the meaning of the present invention is meant to refer to a material which is considered by a person skilled in the art to be safe when being in contact with a living organism (e.g. a human) over a longer period of time (e.g. when used in an implantable medical device).

The "median particle size d₅₀" indicates a diameter value such that 50% of the particles have a diameter of less than this value. A "particle size d₉₀" indicates a diameter value such that 90% of the particles have a diameter of less than this value. Preferably, the particle size as defined herein is measured as a "volume-based" particle size distribution. For example, a "volume-based" median particle size d₅₀ indicates a diameter value such that 50 % by volume of the particles have a diameter of less than this value. The volume-based particle size distribution can be measured by laser diffraction, e.g. using a Malvern Mastersizer 2000 or 3000 laser diffraction system.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the terms "essentially consisting of" and "consisting of" are considered to be a preferred embodiments of the term "comprising". If hereinafter a group is defined to comprise at least a certain number of embodiments, this is also to be understood to disclose a group, which preferably essentially consists of only of these embodiments, or preferably consists of only of these embodiments.

Terms like "obtainable" or "definable" and "obtained" or "defined" are used interchangeably.

This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

### DETAILED DESCRIPTION

### Electrically conductive adhesive

The invention provides an electrically conductive adhesive comprising
a) a (meth)acrylate monomer,
b) a polymer being soluble in the (meth)acrylate monomer, and wherein the polymer is selected from the group consisting of poly((meth)acrylates), poly((meth)acrylate) copolymers, and a mixture thereof,
c) a biocompatible metal having a median particle size d₅₀ of below 50 µm, and
d) a polymerization initiator.

The electrically conductive adhesive according to the present invention comprises a (meth)acrylate monomer. The (meth)acrylate monomer is polymerizable. Thus, the (meth)acrylate monomer shall polymerize when the adhesive is cured, e.g. after its application to a substrate, and initiation of the polymerization. The (meth)acrylate monomer is preferably polymerizable in a radical polymerization. After polymerization and/or curing of the adhesive, the (meth)acrylate, together with other organic compounds of the adhesive, forms the matrix of the adhesive into which the biocompatible metal is embedded.

The (meth)acrylate monomer may be a (meth)acrylate monomer with a boiling point above 90°C, preferably in the range of 90 to 200°C, and more preferably in the range of 95 to 200°C.

The (meth)acrylate monomer may have a molecular weight of below 1000 g/mol, preferably from 90 to 1000 g/mol, more preferably from 90 to 400 g/mol, and most preferably from 100 to 200 g/mol.

The (meth)acrylate monomer and/or the polymer derived from the (meth)acrylate monomer is preferably a biocompatible material, and more preferably a biocompatible material according to ISO 10993.

The (meth)acrylate monomer may be a methacrylate monomer or an acrylate monomer, and preferably the (meth)acrylate monomer is a methacrylate monomer.

The (meth)acrylate monomer may be selected from the group consisting of alkyl (meth)acrylate monomers, diol di(meth)acrylate monomers, and mixtures thereof. It is preferred that the (meth)acrylate monomer is selected from the group consisting of alkyl (meth)acrylate monomers.

The alkyl group of the alkyl (meth)acrylate monomer may be substituted or unsubstituted, but it is preferred that the alkyl group is an unsubstituted alkyl group. The alkyl group of the (meth)acrylate monomer may be saturated or unsaturated, and preferably is saturated. The alkyl group of the (meth)acrylate monomer may be branched or unbranched, and preferably is unbranched. The diol group of the diol di(meth)acrylate may be substituted or unsubstituted, saturated or unsaturated, and branched or unbranched. Preferably, the diol group is unsubstituted, saturated and unbranched.

Preferably, the alkyl (meth)acrylate monomer is a C₁-C₂₀-alkyl (meth)acrylate monomer, more preferably a C₁-C₁₀-alkyl (meth)acrylate monomer, even more preferably a C₁-C₆-alkyl (meth)acrylate monomer, and most preferably a C₁-C₄-alkyl (meth)acrylate monomer.

The diol di(meth)acrylate monomer is preferably a C₁-C₁₀-diol di(meth)acrylate such as ethyleneglycol dimethacrylate, butane-1,4-diol dimethacrylate or hexane-1,6-diol dimethacrylate.

According to one embodiment, the (meth)acrylate monomer is at least one of methyl methacrylate, ethyl methacrylate, ethyleneglycol dimethacrylate and butane-1,4-diol dimethacrylate. According to a preferred embodiment, the (meth)acrylate monomer is a C₁-C₁₀-alkyl methacrylate, preferably a C₁-C₆-alkyl methacrylate, and most preferably is methyl methacrylate (MMA).

The (meth)acrylate monomer may be present in the electrically conductive adhesive in specific amounts. For example, the (meth)acrylate monomer may be present in the adhesive in an amount in the range of 60 to 90 wt.%, preferably of 65 to 80 wt.%, more preferably of 65 to 75 wt.%, and most preferably of 68 to 74 wt.%, based on the total weight of the organic compounds in the adhesive. The "total weight of the organic compounds in the adhesive" is to be understood in that the basis of the indicated weight is the sum of the weight of all organic compounds in the adhesive. Organic compounds in this context are initiators, polymers, monomers, organic crosslinkers, organic primers etc. The biocompatible metal is not an organic compound.

The (meth)acrylate monomer may be present in the adhesive in an amount in the range of 5 to 40 wt.%, preferably of 10 to 30 wt.%, more preferably of 12 to 20 wt.%, based on the total weight of the adhesive. The (meth)acrylate monomer may be present in the adhesive in an amount in the range of 30 to 85 % by volume, preferably of 35 to 75% by volume, more preferably of 40 to 75% by volume, based on the total volume of the adhesive.

The electrically conductive adhesive may also comprise further polymerizable monomers, and preferably radically polymerizable monomers. For example, the electrically conductive monomer may comprise at least one further monomer being different from the monomer described herein as monomer a), wherein the monomer is selected from the group of (meth)acrylate monomers, (meth)acrylic acid, (meth)acrylamide (e.g. methacrylamide), and mixtures thereof. Preferably, the electrically conductive adhesive further comprises a (meth)acrylamide and/or a di(meth)acrylate, and more preferably further comprises methacrylamide and ethyleneglycol dimethacrylate.

The electrically conductive adhesive according to the present invention comprises a polymer being soluble in the (meth)acrylate monomer, and wherein the polymer is selected from the group consisting of poly((meth)acrylates), poly((meth)acrylate) copolymers, and a mixture thereof.

It has been found by the inventors that including the polymer as described herein (herein also referred to as the polymer b)) into the electrically conductive adhesive has several advantages. The polymer may be included into the adhesive to adjust the viscosity and/or internal strength of the adhesive to a suitable value. Furthermore, it has been found that by including the polymer into the adhesive the curing time of the adhesive may be influenced and/or adjusted to a suitable curing time.

The polymer is preferably soluble in the (meth)acrylate monomer in an amount of at least 25 g/L (e.g. in the range of 25 to 600 g/L) at a temperature of 25°C, preferably of at least 50 g/L, and more preferably of at least 100 g/L (e.g. in the range of 100 to 600 g/L).

The polymer is preferably a polymer having a weight median molecular weight of below 750 000 g/mol (e.g. of 250 to 750 000 g/mol), preferably of below 500 000 g/mol, more preferably of below 200 000 g/mol. The weight median molecular weight of the polymer is preferably determined by a viscometric method.

The polymer is preferably a non-crosslinked polymer.

Preferably, the polymer is a biocompatible polymer according to ISO 10993.

The polymer may be a homopolymer or a copolymer. According to the claimed invention, the polymer is selected from the group consisting of poly((meth)acrylates) poly((meth)acrylate) copolymers, and a mixture thereof. More preferably, the polymer is selected from the group consisting of poly(methacrylates) polymethacrylate copolymers, and a mixture thereof. Even more preferably, the polymer is selected from the group consisting of poly(alkyl methacrylates), poly(alkyl methacrylate) copolymers, and a mixture thereof. Yet even more preferably, the polymer is selected from the group consisting of of poly(methyl methacrylates), poly(methyl methacrylate) copolymers, and a mixture thereof.

According to one preferred embodiment, the polymer is selected from the group consisting of poly(methyl methacrylates) (PMMA), poly(ethyl methacrylates) (PEMA), poly(propyl methacrylates), poly(isopropyl methacrylates), poly(methyl methacrylate-co-methacrylate), poly(methyl methacrylate-co-styrene), and mixtures thereof. According to a more preferred embodiment, the polymer is a poly(methyl methacrylate) (PMMA). A suitable poly(methyl methacrylate) is Degacryl^{®} MW 332 from Evonik.

The polymer may be present in the adhesive in specific amounts. For example, the polymer may be present in the adhesive in an amount in the range of 10 to 30 wt.%, preferably 12 to 25 wt.%, more preferably 15 to 22 wt.%, based on the total weight of the organic compounds in the adhesive.

The polymer may be present in the adhesive in an amount in the range of 0.75 to 12 wt.%, preferably 1.5 to 8 wt.%, more preferably 2 to 6 wt.%, based on the total weight of the adhesive. The polymer may be present in the adhesive in an amount in the range of 10 to 30% by volume, preferably of 10 to 25% by volume, based on the total volume of the adhesive.

The electrically conductive adhesive according to the present invention comprises a biocompatible metal having a particle size d₅₀ of below 50 µm. According to a preferred embodiment, the particle size distribution of the metal as indicated herein is a volume-based particle size distribution, which is preferably measured by laser diffraction.

Preferably, the biocompatible metal is a biocompatible metal according to ISO 10993.

According to another embodiment, the biocompatible metal is a biocompatible metal according to ISO 10993, which is not gold.

The biocompatible metal is preferably selected from the group consisting platinum, gold, iridium, steel, titanium, hafnium, niobium, tantalum, cobalt, chromium, zirconium, rhenium, tungsten, molybdenum, and alloys of each one of these metals. More preferably, the biocompatible metal is selected from the group consisting of platinum, iridium, steel, titanium, hafnium, niobium, tantalum, cobalt, chromium, zirconium, rhenium, tungsten, molybdenum, and alloys of each one of these metals.

According to a preferred embodiment, the biocompatible metal is platinum or a platinum alloy (e.g. a platinum/iridium alloy). Most preferably, the biocompatible metal is platinum.

The biocompatible metal preferably has a particle size d₅₀ in the range of from 0.01 to 50 µm, preferably from 0.01 to 25 µm, more preferably from 0.2 to 10 µm, and most preferably from 0.5 to 3 µm. Furthermore, the biocompatible metal preferably has a particle size d₉₀ in the range of from 0.1 to 80 µm, preferably from 0.5 to 40 µm, more preferably from 1.0 to 20 µm, and most preferably from 1.0 to 8 µm. According to one embodiment, the biocompatible metal has a particle size d₅₀ in the range of from 0.2 to 10 µm, preferably from 0.5 to 3 µm, and a particle size d₉₀ in the range of from 1.0 to 20 µm, preferably from 1.0 to 8 µm.

The biocompatible metal may have a specific surface area in the range of 0.01 to 50 m²/g, preferably 0.4 to 30 m²/g, more preferably of 0.5 to 10 m²/g, even more preferably from 0.5 to 5 m²/g, as measured with nitrogen and the BET method.

The biocompatible metal may comprise particles having a specific particle geometry such as spheric, needles or plates. Preferably, the biocompatible metal comprises particles having a needle or flake geometry.

The biocompatible metal is included into the adhesive to render the adhesive electrically conductive. Therefore, it is understood by a skilled person that the biocompatible metal is present in the adhesive in an amount to render the adhesive conductive. The amount of the biocompatible metal which needs to be added to the adhesive to render the adhesive conductive may also be expressed by the percolation threshold. So, the biocompatible metal may be present in the adhesive in an amount above its percolation threshold.

The biocompatible metal may be present in the adhesive in an amount in the range of 50 to 95 wt.%, preferably 60 to 90 wt.%, more preferably 70 to 85 wt.%, and most preferably in an amount of 73 to 78 wt.% (e.g. 76 to 78 wt.%), based on the total weight of the adhesive.

Furthermore, the biocompatible metal may be present in the adhesive in an amount of from 7.5 to 50% by volume, preferably of from 10 to 50% by volume, more preferably of from 12.5 to 50% by volume, even more preferably from 14 to 30% by volume (e.g. from 14 to 20% by volume), based on the total volume of the adhesive.

According to another embodiment, the biocompatible metal is present in the adhesive in an amount of from 20 to 50% by volume, preferably of from 30 to 50% by volume, based on the total volume of the adhesive.

The electrically conductive adhesive according to the invention comprises a polymerization initiator.

The polymerization initiator is included into the adhesive to initiate polymerization of the (meth)acrylate monomer as defined herein. The polymerization initiator is preferably stable at a temperature of 25°C.

The polymerization initiator is preferably a radical polymerization initiator. For example, the initiator may be a peroxide, a diazo compound, a barbiturate, or a photoinitiator. Suitable peroxides are e.g. dibenzoyl peroxide or cumolhydroxy peroxide. Suitable diazo compounds are e.g. azobis(isobutyronitrile) (AIBN) or dimethyl-azobis(2-methylpropionate) (V-601). Suitable barbiturates are e.g. selected from the group of 1,5-disubstituted barbiturates, 1,3,5-trisubstituted barbiturates and 1,3,5-tetrasubstituted barbiturates (e.g. selected from the group of 1-cyclohexyl-5-ethyl barbituric acid, 1-phenyl-5-ethyl barbituric acid, 1-benzyl-5-ethyl barbituric acid and 1,3,5-trimethyl barbituric acid). It is preferred that the initiator is a peroxide, preferably dibenzoyl peroxide.

The polymerization initiator may be activated by adding a polymerization activator to the adhesive, by applying heat to the adhesive, and/or by applying UV light to the adhesive. Preferably, the initiator can be activated by adding a polymerization activator to the adhesive and/or by applying heat to the adhesive. According to one embodiment, the initiator can be activated by applying heat to the adhesive.

The polymerization initiator may be a compound having a self accelerating decomposition temperature (SADT) in the range of 35 to 100°C, preferably in the range of 50 to 85°C.

The polymerization initiator may be present in the adhesive in an amount in the range of from 0.1 to 3.0 wt.%, preferably from 0.5 to 2.5 wt.%, more preferably from 1.2 to 2.4 wt.%, based on the total weight of the organic compounds in the adhesive. Moreover, the polymerization initiator may be present in the adhesive in an amount in the range of from 0.05 to 2.0 wt.%, preferably from 0.1 to 1.5 wt.%, more preferably from 0.2 to 1.0 wt.%, based on the total weight of the adhesive. The polymerization initiator may be present in the adhesive in an amount in the range of from 0.02 to 3.0% by volume, preferably from 0.2 to 2.5% by volume, more preferably from 1.2 to 2.4 wt.%, based on the total volume of the adhesive.

The components may also be present in the adhesive in specific relative amounts to each other. It is preferred that the adhesive comprises the following components in the following relative amounts (with pbw being "parts by weight"):

| | |
|---|---|
| (meth)acrylate monomer | 60 to 100 pbw, preferably 70 to 90 pbw, |
| polymer b) | 10 to 30 pbw, preferably 16 to 24 pbw, |
| initiator | 1.0 to 3.0 pbw, preferably 1.6 to 2.4 pbw. |

In addition to the components above, the electrically conductive adhesive may comprise further components. For example, the electrically conductive adhesive may comprise further polymers (e.g. polymers which are not soluble in the (meth)acrylate monomer), polymerization activators, antibiotics, stabilizers, primers, crosslinkers, tackifying agents, and/or fillers. "A polymer being not soluble in the (meth)acrylate monomer" means that the polymer dissolves with less than 25g/L, preferably less than 5 g/L, in the monomer at a temperature of 25°C.

According to a preferred embodiment of the present invention, the electrically conductive adhesive comprises a primer and/or a crosslinker. According to one preferred embodiment, the adhesive comprises a primer and a crosslinker.

The electrically conductive adhesive preferably comprises a crosslinker. The crosslinker may be any kind of crosslinker which is suitable as a crosslinker in poly((meth)acrylate) adhesives (e.g. difunctional or trifunctional compounds). For example, the crosslinker may be a diol di(meth)acrylate (e.g. ethyleneglycol dimethacrylate, butane-1,4-diol dimethacrylate, or hexane-1,4-diol dimethacrylate). Preferably, the crosslinker is ethyleneglycol dimethacrylate.

Preferably, the crosslinker is present in the adhesive in an amount in the range of 1.0 to 15 wt.%, preferably of 3.0 to 10 wt.%, more preferably of 4 to 8 wt.%, based on the total weight of the organic compounds in the adhesive. According to another preferred embodiment, the adhesive comprises a crosslinker in an amount in the range of 0.1 to 6 wt.%, preferably of 0.4 to 3 wt.%, based on the total weight of the adhesive. According to another embodiment, the crosslinker is present in the adhesive in an amount in the range of 0.5 to 14% by volume, preferably of 1.5 to 10% by volume, based on the total volume of the adhesive.

The electrically conductive adhesive preferably comprises a primer. The primer may be any primer which is suitable for use as a primer in poly((meth)acrylate) adhesives. For example, the primer may be (meth)acrylamide. Preferably, the primer is methacrylamide.

Preferably, the primer is present in the adhesive in an amount in the range of 0.5 to 5.0 wt.%, preferably of 1.5 to 3.5 wt.%, more preferably, of 2.0 to 3.0 wt.%, based on the total weight of the organic compounds in the adhesive. According to another preferred embodiment, the adhesive comprises a primer in an amount in the range of 0.05 to 2.5 wt.%, preferably of 0.1 to 1.2 wt.%, based on the total weight of the adhesive. According to another embodiment, the primer is present in the adhesive in an amount in the range of 0.25 to 14% by volume, preferably of 1.0 to 10% by volume, based on the total volume of the adhesive.

According to another preferred embodiment, the adhesive comprises the following components in the following relative amounts:

| | |
|---|---|
| (meth)acrylate monomer | 60 to 100 pbw, preferably 70 to 90 pbw, |
| polymer b) | 10 to 30 pbw, preferably 16 to 24 pbw, |
| initiator | 1.0 to 3.0 pbw, preferably 1.6 to 2.4 pbw, |
| crosslinker | 3.0 to 9.0 pbw, preferably 5.0 to 7.0 pbw, |
| primer | 0.8 to 4.0 pbw, preferably 2.0 to 3.5 pbw. |

The electrically conductive adhesive may comprise a polymerization activator. The polymerization activator may be an aromatic amine (e.g. N,N-dimethyl-p-toluidine, N,N-bis-hydroxyethyl-p-toluidine, N,N-dimethyl-aniline, 4-N,N-dimethylamino-pyridine), saccharine, lithium chloride, trioctylmethylammonium chlorid, or mixtures thereof. Preferably, the activator is an aromatic amine, and particularly N,N-dimethyl-p-toluidine. The foregoing activators are particularly preferred, if the polymerization initiator is a peroxide.

The polymerization activator may also be a copper(ll) salt, preferably selected from the group of copper(II) ethyl hexanoate, copper(ll) methacrylate, copper(ll) acetyl acetonate, basic copper(ll) carbonate, copper(II) hydroxide, and mixtures thereof. Preferably, the polyimerization activator is a copper(II) salt, if the polymerization initiator is a barbiturate.

The electrically conductive may comprise a polymerization activator in an amount of 0.05 to 3.0 wt.%, preferably from 0.2 to 2.0 wt.%, based on the total weight of the organic compounds in the adhesive. According to one embodiment, the polymerization activator is present in the adhesive in an amount in the range of from 0.01 to 1.0 wt.%, preferably from 0.02 to 0.5 wt.%, more preferably from 0.04 to 0.2 wt.%, based on the total weight of the adhesive. The polymerization initiator may be present in the adhesive in an amount in the range of from 0.01 to 1.5% by volume, preferably from 0.1 to 1.2% by volume, more preferably from 0.6 to 1.0 wt.%, based on the total volume of the adhesive.

The polymerization activator is preferably used in a specific relative amount with respect to the polymerization initiator. The polymerization activator is preferably used in a weight ratio to the polymerization initiator in the range of 1:10 to 2:1 [activator:initiator], preferably in the range of 1:6 to 1.5:1, more preferably in the range of 1:4 to 1:1.

The electrically conductive adhesive may have an electrical resistance of below 20 Ohm, preferably in the range of 0.05 to 10 Ohm, more preferably in the range of 0.1 to 5 Ohm, even more preferably in the range of 0.1 to 2 Ohm, and most preferably in the range of 0.1 to 1 Ohm. The electrical resistance is measured for the cured adhesive e.g. by using a Mulimeter Agilent 34401A.

Preferably, the electrically conductive adhesive is an isotropic conductive adhesive.

According to one very preferred embodiment, the electrically conductive adhesive comprises
a) a C₁-C₁₀-alkyl methacrylate, preferably methyl methacrylate (MMA),
   wherein the C₁-C₁₀-alkyl methacrylate is present in the adhesive in an amount of in an amount in the range of 5 to 40 wt.%, preferably of 10 to 30 wt.%, based on the total weight of the adhesive,
b) a polymer being soluble in the (meth)acrylate monomer, which is selected from the group consisting of poly((meth)acrylates), poly((meth)acrylate) copolymers, and a mixture thereof, preferably is poly(methyl methacrylate) (PMMA),
   wherein the polymer is present in the adhesive in amount in the range of 0.75 to 12 wt.%, preferably 1.5 to 8 wt.%, based on the total weight of the adhesive,
c) a biocompatible metal having a median particle size d₅₀ of below 50 µm, which is selected from the group consisting of platinum, gold, iridium, steel, titanium, hafnium, niobium, tantalum, cobalt, chromium, zirconium, rhenium, tungsten, molybdenum, and alloys of each one of these metals, preferably is platinum,
   wherein the biocompatible metal is present in the adhesive in an amount in the range of 50 to 85 wt.%, preferably 70 to 82 wt.%, and more preferably 75 to 80 wt.%, based on the total weight of the adhesive,
d) a polymerization initiator, which is selected from the group consisting of peroxides, barbiturates, and mixture thereof, preferably a peroxide,
   wherein the polymerization initiator is present in the adhesive in an amount in the range of from 0.02 to 1.5 wt.%, preferably from 0.1 to 1.0 wt.%, based on the total weight of the adhesive,
e) a crosslinker, preferably selected from the group consisting of diol di(meth)acrylates,
   wherein the crosslinker is present in the adhesive in an amount in the range of 0.1 to 6 wt.%, preferably of 0.4 to 3 wt.%, based on the total weight of the adhesive,
f) a primer, preferably methacrylamide,
   wherein the primer is present in the adhesive in an amount in the range of 0.05 to 2.5 wt.%, preferably of 0.1 to 1.2 wt.%, based on the total weight of the adhesive.

The electrically conductive adhesive may have a curing time of below 30 min, preferably in the range of 1 to 20 min, more preferably in the range of 2 to 15 min.

According to a preferred embodiment, the electrically conductive adhesive is suitable for being used to connect electronic parts in an implantable medical device. Preferably, the implantable medical device is an active implantable medical device (AIMD). Preferred AIMDs are, for example, cardiac pacemakers, cardiac defibrillators, neurostimulators, cochlea implants, implantable cardioverters, nerve, brain, organ or muscle stimula tors as well as implantable monitoring devices, hearing aids, retinal implants, muscle stimulators, implantable drug pumps, artificial hearts, bone growth stimulators, prostate implants and stomach implants.

### Kit for an electrically conductive adhesive

Another aspect of the present invention relates to a kit for preparing an electrically conductive adhesive. The kit comprises a component A and a component B,
wherein component A comprises a (meth)acrylate monomer, and
wherein component B comprises a polymer being soluble in the (meth)acrylate monomer of component A, and wherein the polymer is selected from the group consisting of poly((meth)acrylates), poly((meth)acrylate) copolymers, and a mixture thereof, a biocompatible metal having a median particle size d₅₀ of below 50 µm, and a polymerization initiator.

The inventive kit can be used to prepare an electrically conductive adhesive according the present invention by mixing component A with component B. According to a preferred embodiment, the kit is a kit for preparing an electrically conductive adhesive as defined in the foregoing section.

The foregoing section discloses embodiments, and preferred embodiments, for the (meth)acrylate monomer of the inventive adhesive. The same embodiments, and preferred embodiments, are also disclosed herein in connection with and/or also apply to the (meth)acrylate monomer of component A of the inventive kit. This means that an embodiment of the (meth)acrylate monomer, which is described in the foregoing section, is also an embodiment of the (meth)acrylate monomer of component A of the inventive kit.

The (meth)acrylate monomer is present in component A of the kit. Optionally, the (meth)acrylate monomer is also present in component B of the kit. For example, the (meth)acrylate monomer may be present in component B of the kit, if it is desired that component B is not a solid such as a powder but a paste.

The (meth)acrylate monomer may be present in the kit in specific amounts. For example, the (meth)acrylate monomer may be present in (e.g. in component A) in an amount in the range of 60 to 90 wt.%, preferably of 65 to 80 wt.%, more preferably of 65 to 75 wt.%, and most preferably of 68 to 74 wt.%, based on the total weight of the organic compounds of components A and B. The (meth)acrylate monomer may be present in the kit (e.g. in component A) in an amount in the range of 5 to 40 wt.%, preferably of 10 to 30 wt.%, more preferably of 12 to 20 wt.%, based on the total weight of all components of the kit. The (meth)acrylate monomer may be present in (e.g. in component A) in an amount in the range of 30 to 85 % by volume, preferably of 35 to 75% by volume, more preferably of 40 to 75% by volume, based on the total volume of all components of the kit.

The foregoing section discloses embodiments, and preferred embodiments, for the polymer, the biocompatible metal powder, and the polymerization initiator of the inventive adhesive. The same embodiments, and preferred embodiments, are also disclosed herein in connection with and/or also apply to the polymer, the biocompatible metal, and the polymerization initiator of component B of the inventive kit. This means that an embodiment of the polymer, the biocompatible metal, or the polymerization initiator, which is described in the foregoing section, is also an embodiment of the polymer, the biocompatible metal, or the polymerization initiator of component B of the inventive kit.

The polymer is present in component B of the kit. Optionally, the polymer is also present in component A of the kit. For example, the polymer may be present in component A of the kit to adjust the viscosity of component A e.g. to a paste-like viscosity.

The polymer may be present in the kit in specific amounts. For example, the polymer may be present in the kit (e.g. in component B) in an amount in the range of 10 to 30 wt.%, preferably 12 to 25 wt.%, more preferably 15 to 22 wt.%, based on the total weight of the organic compounds of components A and B of the kit. The polymer may be present in the kit (e.g. in component B) in an amount in the range of 0.75 to 12 wt.%, preferably 1.5 to 8 wt.%, more preferably 2 to 6 wt.%, based on the total weight of components A and B of the kit. The polymer may be present in the kit (e.g. in component B) in an amount in the range of 10 to 30% by volume, preferably of 10 to 25% by volume, based on the total volume of components A and B of the kit.

The biocompatible metal may be present in the kit (e.g. in component B) in an amount in the range of 50 to 95 wt.%, preferably 60 to 90 wt.%, more preferably 70 to 85 wt.%, and most preferably in an amount of 73 to 78 wt.% (e.g. 76 to 78 wt.%), based on the total weight of components A and B of the kit. Furthermore, the biocompatible metal may be present in the kit (e.g. in component B) in an amount of from 7.5 to 50% by volume, preferably of from 10 to 50% by volume, more preferably of from 12.5 to 50% by volume, even more preferably from 14 to 30% by volume (e.g. from 14 to 20% by volume), based on the total volume of components A and B of the kit. According to another embodiment, the biocompatible metal is present in the kit (e.g. component B) in an amount of from 20 to 50% by volume, preferably of from 30 to 50% by volume, based on the total volume of components A and B of the kit.

The polymerization initiator may be present in the kit (e.g. in component B) in an amount in the range of from 0.1 to 3.0 wt.%, preferably from 0.5 to 2.5 wt.%, more preferably from 1.2 to 2.4 wt.%, based on the total weight of the organic compounds of components A and B of the kit. Moreover, the polymerization initiator may be present in the kit (e.g. in component B) in an amount in the range of from 0.05 to 2.0 wt.%, preferably from 0.1 to 1.5 wt.%, more preferably from 0.2 to 1.0 wt.%, based on the total weight of components A and B of the kit. The polymerization initiator may be present in the kit (e.g. in component B) in an amount in the range of from 0.02 to 3.0% by volume, preferably from 0.2 to 2.5% by volume, more preferably from 1.2 to 2.4 wt.%, based on the total volume of components A and B of the kit.

Components A and/or B of the kit may further comprise further polymers, polymerization activators, antibiotics, stabilizers, primers, crosslinkers, tackifying agents, and/or fillers. Preferred polymerization activators, primers and crosslinkers are described in the foregoing section with respect to the inventive electrically conductive adhesive.

According to a preferred embodiment, component A of the kit comprises a polymerization activator. Preferred polymerization activator are described in the foregoing section with respect to the inventive electrically conductive adhesive.

The kit (e.g. component A) may comprise a polymerization activator in an amount of 0.05 to 3.0 wt.%, preferably from 0.2 to 2.0 wt.%, based on the total weight of the organic compounds in components A and B of the kit. According to one embodiment, the polymerization activator is present in the kit (e.g. component A) in an amount in the range of from 0.01 to 1.0 wt.%, preferably from 0.02 to 0.5 wt.%, more preferably from 0.04 to 0.1 wt.%, based on the total weight of components A and B of the kit. The polymerization initiator may be present in the kit (e.g. component A) in an amount in the range of from 0.01 to 1.5% by volume, preferably from 0.1 to 1.2% by volume, more preferably from 0.6 to 1.0 wt.%, based on the total volume of components A and B of the kit.

According to a preferred embodiment, component A of the kit further comprises a crosslinker and/or a primer, preferably a primer and a crosslinker.

Preferably, the crosslinker is present in the kit (e.g. in component A) in an amount in the range of 1.0 to 15 wt.%, preferably of 3.0 to 10 wt.%, more preferably of 4 to 8 wt.%, based on the total weight of the organic compounds in components A and B of the kit. According to another preferred embodiment, the kit (e.g. in component A) comprises a crosslinker in an amount in the range of 0.1 to 6 wt.%, preferably of 0.4 to 3 wt.%, based on the total weight of components A and B of the kit. According to another embodiment, the crosslinker is present in the kit (e.g. in component A) in an amount in the range of 0.5 to 14% by volume, preferably of 1.5 to 10% by volume, based on the total volume of components A and B.

Preferably, the primer is present in the kit (e.g. in component A) in an amount in the range of 0.5 to 5.0 wt.%, preferably of 1.5 to 3.5 wt.%, more preferably, of 2.0 to 3.0 wt.%, based on the total weight of the organic compounds in components A and B of the kit. According to another preferred embodiment, the kit (e.g. in component A) comprises a primer in an amount in the range of 0.05 to 2.5 wt.%, preferably of 0.1 to 1.2 wt.%, based on the total weight of components A and B of the kit. According to another embodiment, the primer is present in the kit (e.g. in component A) in an amount in the range of 0.25 to 14% by volume, preferably of 1.0 to 10% by volume, based on the total volume of components A and B of the kit.

According to a preferred embodiment, the kit is a kit for preparing an electrically conductive adhesive, which is suitable for being used to connect electronic parts in an implantable medical device. Preferably, the implantable medical device is an active implantable medical device (AIMD). Preferred AIMDs are, for example, cardiac pacemakers, cardiac defibrillators, neurostimulators, cochlea implants, implantable cardioverters, nerve, brain, organ or muscle stimula tors as well as implantable monitoring devices, hearing aids, retinal implants, muscle stimulators, implantable drug pumps, artificial hearts, bone growth stimulators, prostate implants and stomach implants.

### Implantable medical device

In another aspect of the present invention, an implantable medical device is provided. The implantable medical device comprises at least two electronic parts,
wherein the at least two electronic parts are connected by
a) the electrically conductive adhesive according to the present invention, or a cured form thereof, or
b) the electrically conductive adhesive prepared from the kit according to the present invention, or a cured form thereof.

A skilled person understands that a cured form of the electrically conductive adhesive of the present invention comprises a polymer which is derived from the (meth)acrylate monomer of the adhesive. The cured form of the adhesive further comprises the biocompatible metal, which renders the cured form of the adhesive electrically conductive. The cured form of the adhesive further comprises the polymer of the inventive adhesive, or a reaction product thereof. If at all, the cured form of the adhesive only comprises the polymerization initiator in a significantly lower amount than the non-cured form of the adhesive. According to a preferred embodiment, the at least two electronic parts are connected by a cured form of the electrically conductive adhesive.

According to one preferred embodiment, the at least two electronic parts are connected by the electrically conductive adhesive according to the present invention, or a cured form thereof. According to another embodiment, the at least two electronic parts are connected by the electrically conductive adhesive prepared from the kit according to the present invention, or a cured form thereof. According to a preferred embodiment, the at least two electronic parts are connected by a cured form of the electrically conductive adhesive.

According to a preferred embodiment, the implantable medical device is an active implantable medical device (AIMD). Preferred AIMDs are, for example, cardiac pacemakers, cardiac defibrillators, neurostimulators, cochlea implants, implantable cardioverters, nerve, brain, organ or muscle stimulators as well as implantable monitoring devices, hearing aids, retinal implants, muscle stimulators, implantable drug pumps, artificial hearts, bone growth stimulators, prostate implants and stomach implants.

The at least two electronic parts are not particularly limited. The at least two electronic parts may be independently selected from the group of wires, electrical feedthroughs (e.g. cermet feedthroughs), electrodes, microelectrodes, electrode arrays, flexible electrode arrays, electronic leads, electronic terminals, and contact elements.

According to one preferred embodiment, the at least two electronic parts are a wire and an electrode array (e.g. a flexible electrode array). According to another preferred embodiment, the at least two electronic parts are a wire and an electrical feedthrough (e.g. cermet feedthrough). A "cermet feedthrough" is a feedthrough comprising a ceramic component and a metal component. Such feedthroughs are known to the skilled person. According to yet another preferred embodiment, the at least two electronic parts are an electrode (e.g. a microelectrode) and a lead.

### Use and/or process

Another aspect of the present invention relates to a use of an electrically conductive adhesive according to the present invention, or use of a kit according to the present invention, for connecting at least two electronic parts in an implantable medical device.

Thus, one embodiment refers to the use of a kit according to the present invention for connecting at least two electronic parts in an implantable medical device. One preferred embodiment refers to a use of an electrically conductive adhesive according to the present invention for connecting at least two electronic parts in an implantable medical device. According to a preferred embodiment, the implantable medical device is an active implantable medical device (AIMD). Preferred AIMDs are, for example, cardiac pacemakers, cardiac defibrillators, neurostimulators, cochlea implants, implantable cardioverters, nerve, brain, organ or muscle stimulators as well as implantable monitoring devices, hearing aids, retinal implants, muscle stimulators, implantable drug pumps, artificial hearts, bone growth stimulators, prostate implants and stomach implants.

The at least two electronic parts are not particularly limited. The at least two electronic parts may be independently selected from the group of wires, electrical feedthroughs (e.g. cermet feedthroughs), electrodes, microelectrodes, electrode arrays, flexible electrode arrays, electronic leads, electronic terminals, and contact elements.

According to one preferred embodiment, the at least two electronic parts are a wire and an electrode array (e.g. a flexible electrode array). According to another preferred embodiment, the at least two electronic parts are a wire and an electrical feedthrough (e.g. cermet feedthrough). A "cermet feedthrough" is a feedthrough comprising a ceramic component and a metal component. Such feedthroughs are known to the skilled person. According to yet another preferred embodiment, the at least two electronic parts are an electrode (e.g. a microelectrode) and a lead.

Also described herein is a process for connecting at least two electronic parts in an implantable medical device by using of the electrically conductive adhesive according to the present invention, or by using the kit according to the present invention.

According to a preferred embodiment, the process uses the electrically conductive adhesive according to the present invention. In such case, the process may comprise the steps of 1) applying the electrically conductive adhesive according to the present invention to a first electronic part, 2) contacting the applied electrically conductive adhesive to the second electronic part, and 3) curing the electrically conductive adhesive.

According to a preferred embodiment, the process uses the kit according to the present invention. In such case, the process may comprise the steps of 1) mixing component A with component B of the kit to prepare an electrically conductive adhesive, 2) applying the electrically conductive adhesive to a first electronic part, 3) contacting the applied electrically conductive adhesive to the second electronic part, and 4) curing the electrically conductive adhesive.

The features disclosed in the claims and the specification may be essential for different embodiments of the claimed invention, both separately and in any combination with each other. In the following, the claimed invention is further described by an example. The example is not understood to be limiting the claimed invention in any way.

### Example

**Table 1: Materials**

| **(Meth)acrylate monomer** | Methyl methacrylate (CAS: 80-62-6) |
|---|---|
| **Polymer (soluble in the monomer)** | Polymethyl methacrylate (PMMA), Degacryl^{®} MW 332, commercially available from Evonik |
| **Biocompatible metal** | Platinum powder, Pt M579B, commercially available from Heraeus, surface area 0.8 - 2.0 m²/g; tap density 3.0 - 4.0; particle size: d₉₀ = 1.5 - 3.0; d₅₀ = 0.9 - 1.9, d₁₀ = 0.5 - 0.9. |
| **Polymerization initiator** | Dibenzoyl peroxide (CAS: 94-36-0) |
| **Polymerization activator** | Dimethyl-p-toluidine (CAS: 99-97-8) |
| **Crosslinker** | Ethyleneglycol dimethacrylate (CAS: 97-90-5) |
| **Primer** | Methacrylamide (CAS: 79-39-0) |
| | |
| **Substrate** | Silver-coated polyimide |

A PMMA solution was prepared by mixing the components as indicated in Table 2 with each other, and stirring the mixture over night.

**Table 2: PMMA solution**

| **Methyl methacrylate (wt.%)** | **PMMA (wt.%)** | **Dibenzoyl peroxide (wt.%)** | **Methacrylamide (wt.%)** | **Ethyleneglycol dimethacrylate (wt.%)** |
|---|---|---|---|---|
| 72.8 | 18 | 1.8 | 2.4 | 6 |

Table 3 shows the recipe of a conductive adhesive according to the invention. The inventive adhesive was prepared by mixing the metal powder into the PMMA solution to obtain a paste, followed by adding the polymerization activator to the mixture. The paste was handled and/or applied manually by using a spatula.

**Table 3: Inventive adhesive**

| **Metal powder (wt.%)** | **PMMA solution (wt.%)** | **Activator (wt.%)** |
|---|---|---|
| 77.98 | 21.98 | 0.04 |

The inventive adhesive was analyzed for its curing time, electrical resistance and its lap shear strength on a silver-coated polyimide substrate. The results are shown in Table 4.

**Table 4: Properties of inventive adhesive**

| **Curing time (min)** | **Lap shear strength according to DIN EN 53 283 (N/mm²)** | **Electrical resistance (Ω)** | **Electrical resistance Ø(10 measurements)** |
|---|---|---|---|
| 7 | 5.63 (failure of silver coating on polyimide) | 0.5 to 1.5 | 1 |

The conductive adhesive showed a lap shear strength according to DIN EN 53 283 of at least 5.63 N/mm². At this shear strength, the silver coating on the polyimide substrate failed so that the actual shear strength of the adhesive is presumably higher than the measured value.

The electrical resistance is an indicator for the electrical conductivity of the adhesive. The average electrical resistance of the adhesive was 1 Ω, which indicates a good electrical conductivity.

## Claims

1. An electrically conductive adhesive comprising
a) a (meth)acrylate monomer,
b) a polymer being soluble in the (meth)acrylate monomer, and wherein the polymer is selected from the group consisting of poly((meth)acrylates), poly((meth)acrylate) copolymers, and a mixture thereof,
c) a biocompatible metal having a median particle size d₅₀ of below 50 µm, and
d) a polymerization initiator.

2. The electrically conductive adhesive according to claim 1, wherein the (meth)acrylate monomer is a C₁-C₁₀-alkyl methacrylate,
and preferably is methyl methacrylate (MMA).

3. The electrically conductive adhesive according to claim 1 or 2, wherein the polymer is selected from the group consisting of poly(methyl methacrylates) (PMMA), poly(ethyl methacrylates) (PEMA), poly(propyl methacrylates), poly(isopropyl methacrylates), poly(methyl methacrylate-co-methacrylate), poly(methyl methacrylate-co-styrene), and mixtures thereof.

4. The electrically conductive adhesive according to any one of the preceding claims, wherein the biocompatible metal is selected from the group consisting of platinum, gold, iridium, steel, titanium, hafnium, niobium, tantalum, cobalt, chromium, zirconium, rhenium, tungsten, molybdenum, and alloys of each one of these metals,
preferably the biocompatible metal is a platinum or a platinum alloy.

5. The electrically conductive adhesive according to any one of the preceding claims, wherein the biocompatible metal has a particle size d₅₀ in the range of from 0.2 to 10 µm, preferably from 0.5 to 3 µm, and/or
wherein the biocompatible metal has a particle size d₉₀ in the range of from 1.0 to 20 µm, preferably from 1.0 to 8 µm.

6. The electrically conductive adhesive according to any one of the preceding claims, wherein the biocompatible metal is present in the adhesive in an amount in the range of 50 to 95 wt.%, preferably 60 to 90 wt.%, and more preferably 70 to 85 wt.%, based on the total weight of the adhesive, and/or
wherein the biocompatible metal is present in the adhesive in an amount of from 7.5 to 50% by volume, preferably of from 10 to 50% by volume, more preferably of from 12.5 to 50% by volume, based on the total volume of the adhesive.

7. The electrically conductive adhesive according to any one of the preceding claims, wherein the (meth)acrylate monomer is present in the adhesive in an amount in the range of 60 to 90 wt.%, preferably of 65 to 75 wt.%, based on the total weight of the organic compounds in the adhesive, and/or
wherein the polymer is present in the adhesive in an amount in the range of 10 to 30 wt.%, preferably 12 to 25 wt.%, based on the total weight of the organic compounds in the adhesive, and/or
wherein the polymerization initiator is present in the adhesive in an amount in the range of from 0.1 to 3.0 wt.%, preferably from 0.5 to 2.5 wt.%, based on the total weight of the organic compounds in the adhesive.

8. The electrically conductive adhesive according to any one of the preceding claims, wherein the adhesive comprises a primer, preferably in an amount in the range of 0.5 to 5.0 wt.%, preferably of 1.5 to 3.5 wt.%, based on the total weight of the organic compounds in the adhesive.

9. The electrically conductive adhesive according to any one of the preceding claims, wherein the adhesive comprises a crosslinker, preferably in an amount in the range of 1.0 to 15 wt.%, preferably of 3.0 to 10 wt.%, based on the total weight of the organic compounds in the adhesive.

10. The electrically conductive adhesive according to any one of the preceding claims, wherein the adhesive comprises a polymerization activator, preferably in an amount of 0.05 to 3.0 wt.%, preferably from 0.2 to 2.0 wt.%, based on the total weight of the organic compounds in the adhesive.

11. The electrically conductive adhesive according to any one of the preceding claims, wherein the polymerization initiator is a compound having a self accelerating decomposition temperature (SADT) in the range of 35 to 100°C, preferably in the range of 50 to 85°C, and/or
wherein the polymerization initiator is a peroxide, a diazo compound, a barbiturate, or a photoinitiator, and preferably is a peroxide.

12. The electrically conductive adhesive according to any one of the preceding claims, wherein the adhesive comprises the following components in the following relative amounts:
| | |
|---|---|
| (meth)acrylate monomer | 60 to 100 pbw, preferably 70 to 90 pbw, |
| polymer b) | 10 to 30 pbw, preferably 16 to 24 pbw, |
| initiator | 1.0 to 3.0 pbw, preferably 1.6 to 2.4 pbw, |
| optionally a crosslinker | 3.0 to 9.0 pbw, preferably 5.0 to 7.0 pbw, |
| optionally a primer | 0.8 to 4.0 pbw, preferably 2.0 to 3.5 pbw. |

13. A kit for preparing an electrically conductive adhesive, preferably an electrically conductive adhesive according to any one of claims 1 to 12, wherein the kit comprises a component A and a component B,
wherein component A comprises a (meth)acrylate monomer, and
wherein component B comprises a polymer being soluble in the (meth)acrylate monomer of component A, and wherein the polymer is selected from the group consisting of poly((meth)acrylates), poly((meth)acrylate) copolymers, and a mixture thereof, a biocompatible metal having a median particle size d₅₀ of below 50 µm, and a polymerization initiator.

14. An implantable medical device comprising at least two electronic parts,
wherein the at least two electronic parts are connected by
a) the electrically conductive adhesive according to any one of claims 1 to 12, or a cured form thereof, or
b) the electrically conductive adhesive prepared from the kit according to claim 13, or a cured form thereof.

15. Use of an electrically conductive adhesive according to any one of claims 1 to 12, or use of a kit according to claim 13, for connecting at least two electronic parts in an implantable medical device.

## Patentansprüche

1. Elektrisch leitfähiger Klebstoff, umfassend
a) ein (Meth)acrylatmonomer,
b) ein Polymer, das in dem (Meth)acrylatmonomer löslich ist, und wobei das Polymer aus der Gruppe ausgewählt ist, bestehend aus Poly((meth)acrylaten), Poly((meth)acrylat)-Copolymeren und einer Mischung davon,
c) ein biokompatibles Metall, das eine mittleren Teilchengröße dso von unter 50 µm aufweist und
d) einen Polymerisationsinitiator.

2. Elektrisch leitfähiger Klebstoff nach Anspruch 1, wobei das (Meth)acrylatmonomer ein C₁-C₁₀-Alkylmethacrylat ist,
und vorzugsweise Methylmethacrylat (MMA) ist.

3. Elektrisch leitfähiger Klebstoff nach Anspruch 1 oder 2, wobei das Polymer aus der Gruppe ausgewählt ist, bestehend aus Poly(methylmethacrylaten) (PMMA), Poly(ethylmethacrylaten) (PEMA), Poly(propylmethacrylaten), Poly(isopropylmethacrylaten), Poly(methylmethacrylat-co-methacrylat), Poly(methylmethacrylat-co-styrol) und Mischungen davon.

4. Elektrisch leitfähiger Klebstoff nach einem der vorstehenden Ansprüche, wobei das biokompatible Metall aus der Gruppe ausgewählt ist, bestehend aus Platin, Gold, Iridium, Stahl, Titan, Hafnium, Niob, Tantal, Kobalt, Chrom, Zirkonium, Rhenium, Wolfram, Molybdän und Legierungen jedes dieser Metalle,
vorzugsweise das biokompatible Metall ein Platin oder eine Platinlegierung ist.

5. Elektrisch leitfähiger Klebstoff nach einem der vorstehenden Ansprüche,
wobei das biokompatible Metall eine Teilchengröße d₅₀ in dem Bereich von 0,2 bis 10 µm, vorzugsweise von 0,5 bis 3 µm, aufweist und/oder
wobei das biokompatible Metall eine Teilchengröße d₉₀ in dem Bereich von 1,0 bis 20 µm, vorzugsweise von 1,0 bis 8 µm, aufweist.

6. Elektrisch leitfähiger Klebstoff nach einem der vorstehenden Ansprüche, wobei das biokompatible Metall in dem Klebstoff in einer Menge in dem Bereich von 50 bis 95 Gew.-%, vorzugsweise 60 bis 90 Gew.-% und mehr bevorzugt 70 bis 85 Gew.-%, basierend auf dem Gesamtgewicht des Klebstoffs, vorhanden ist und/oder
wobei das biokompatible Metall in dem Klebstoff in einer Menge von 7,5 bis 50 Vol.-%, vorzugsweise von 10 bis 50 Vol.-%, mehr bevorzugt von 12,5 bis 50 Vol.-%, basierend auf dem Gesamtvolumen des Klebstoffs, vorhanden ist.

7. Elektrisch leitfähiger Klebstoff nach einem der vorstehenden Ansprüche, wobei das (Meth)acrylatmonomer in dem Klebstoff in einer Menge in dem Bereich von 60 bis 90 Gew.-%, vorzugsweise von 65 bis 75 Gew.-%, basierend auf dem Gesamtgewicht der organischen Verbindungen in dem Klebstoff, vorhanden ist und/oder
wobei das Polymer in dem Klebstoff in einer Menge in dem Bereich von 10 bis 30 Gew.-%, vorzugsweise 12 bis 25 Gew.-%, basierend auf dem Gesamtgewicht der organischen Verbindungen in dem Klebstoff, vorhanden ist und/oder
wobei der Polymerisationsinitiator in dem Klebstoff in einer Menge in dem Bereich von 0,1 bis 3,0 Gew.-%, vorzugsweise von 0,5 bis 2,5 Gew.-%, basierend auf dem Gesamtgewicht der organischen Verbindungen in dem Klebstoff, vorhanden ist.

8. Elektrisch leitfähiger Klebstoff nach einem der vorstehenden Ansprüche, wobei der Klebstoff einen Primer, vorzugsweise in einer Menge in dem Bereich von 0,5 bis 5,0 Gew.-%, vorzugsweise von 1,5 bis 3,5 Gew.-%, basierend auf dem Gesamtgewicht der organischen Verbindungen in dem Klebstoff, umfasst.

9. Elektrisch leitfähiger Klebstoff nach einem der vorstehenden Ansprüche, wobei der Klebstoff einen Vernetzer, vorzugsweise in einer Menge in dem Bereich von 1,0 bis 15 Gew.-%, vorzugsweise von 3,0 bis 10 Gew.-%, basierend auf dem Gesamtgewicht der organischen Verbindungen in dem Klebstoff, umfasst.

10. Elektrisch leitfähiger Klebstoff nach einem der vorstehenden Ansprüche, wobei der Klebstoff einen Polymerisationsaktivator, vorzugsweise in einer Menge von 0,05 bis 3,0 Gew.-%, vorzugsweise von 0,2 bis 2,0 Gew.-%, basierend auf dem Gesamtgewicht der organischen Verbindungen in dem Klebstoff, umfasst.

11. Elektrisch leitfähiger Klebstoff nach einem der vorstehenden Ansprüche, wobei der Polymerisationsinitiator eine Verbindung ist, die eine selbstbeschleunigende Zersetzungstemperatur (SADT) in dem Bereich von 35 bis 100 °C, vorzugsweise in dem Bereich von 50 bis 85 °C, aufweist und/oder wobei der Polymerisationsinitiator ein Peroxid, eine Diazoverbindung, ein Barbiturat oder ein Fotoinitiator ist und vorzugsweise ein Peroxid ist.

12. Elektrisch leitfähiger Klebstoff nach einem der vorstehenden Ansprüche, wobei der Klebstoff die folgenden Komponenten in den folgenden relativen Mengen umfasst:
| | |
|---|---|
| (Meth)acrylatmonomer | 60 bis 100 pbw, vorzugsweise 70 bis 90 pbw, |
| Polymer b) | 10 bis 30 pbw, vorzugsweise 16 bis 24 pbw, |
| Initiator | 1,0 bis 3,0 pbw, vorzugsweise 1,6 bis 2,4 pbw, |
| optional einen Vernetzer | 3,0 bis 9,0 pbw, vorzugsweise 5,0 bis 7,0 pbw, |
| optional einen Primer | 0,8 bis 4,0 pbw, vorzugsweise 2,0 bis 3,5 pbw. |

13. Kit zum Herstellen eines elektrisch leitfähigen Klebstoffs, vorzugsweise eines elektrisch leitfähigen Klebstoffs nach einem der Ansprüche 1 bis 12, wobei das Kit eine Komponente A und eine Komponente B umfasst,
wobei Komponente A ein (Meth)acrylatmonomer umfasst, und
wobei Komponente B umfasst
ein Polymer, das in dem (Meth)acrylatmonomer von Komponente A löslich ist, und wobei das Polymer aus der Gruppe ausgewählt ist, bestehend aus Poly((meth)acrylaten), Poly((meth)acrylat)-Copolymeren und einer Mischung davon,
ein biokompatibles Metall, das eine mittlere Teilchengröße dso von unter 50 µm aufweist und
einen Polymerisationsinitiator.

14. Implantierbare medizinische Vorrichtung, umfassend mindestens zwei elektronische Teile, wobei die mindestens zwei elektronischen Teile verbunden sind durch
a) den elektrisch leitfähigen Klebstoff nach einem der Ansprüche 1 bis 12 oder eine gehärtete Form davon, oder
b) den elektrisch leitfähigen Klebstoff, der aus dem Kit nach Anspruch 13 hergestellt ist, oder eine gehärtete Form davon.

15. Verwendung eines elektrisch leitfähigen Klebstoffs nach einem der Ansprüche 1 bis 12 oder Verwendung eines Kits nach Anspruch 13 zum Verbinden von mindestens zwei elektronischen Teilen in einer implantierbaren medizinischen Vorrichtung.

## Revendications

1. Adhésif électroconducteur comprenant
a) un monomère (méth)acrylate,
b) un polymère étant soluble dans le monomère (méth)acrylate, et dans lequel le polymère est choisi dans le groupe constitué par les poly((méth)acrylates), les copolymères de poly((méth)acrylate) et un mélange de ceux-ci,
c) un métal biocompatible ayant une taille médiane de particules d₅₀ inférieure à 50 µm, et
d) un initiateur de polymérisation.

2. Adhésif électroconducteur selon la revendication 1, dans lequel le monomère (méth)acrylate est un méthacrylate d'alkyle en C₁ à C₁₀,
et de préférence est le méthacrylate de méthyle (MMA).

3. Adhésif électroconducteur selon la revendication 1 ou 2, dans lequel le polymère est choisi dans le groupe constitué par les poly(méthacrylates de méthyle) (PMMA), les poly(méthacrylates d'éthyle) (PEMA), les poly(méthacrylates de propyle), les poly(méthacrylates d'isopropyle), le poly(méthacrylate de méthyle-co-méthacrylate), le poly(méthacrylate de méthyle-co-styrène) et des mélanges de ceux-ci.

4. Adhésif électroconducteur selon l'une quelconque des revendications précédentes, dans lequel le métal biocompatible est choisi dans le groupe constitué par le platine, l'or, l'iridium, l'acier, le titane, l'hafnium, le niobium, le tantale, le cobalt, le chrome, le zirconium, le rhénium, le tungstène, le molybdène, et des alliages de chacun des ces métaux,
de préférence le métal biocompatible est un platine ou un alliage de platine.

5. Adhésif électroconducteur selon l'une quelconque des revendications précédentes, dans lequel le métal biocompatible a une taille de particules dso dans la plage allant de 0,2 à 10 µm, de préférence de 0,5 à 3 µm, et/ou dans lequel le métal biocompatible a une taille de particules d₉₀ dans la plage allant de 1,0 à 20 µm, de préférence de 1,0 à 8 µm.

6. Adhésif électroconducteur selon l'une quelconque des revendications précédentes, dans lequel le métal biocompatible est présent dans l'adhésif en une quantité dans la plage de 50 à 95 % en poids, de préférence 60 à 90 % en poids, et plus préférablement 70 à 85 % en poids, sur la base du poids total de l'adhésif, et/ou
dans lequel le métal biocompatible est présent dans l'adhésif en une quantité allant de 7,5 à 50 % en volume, de préférence allant de 10 à 50 % en volume, plus préférablement allant de 12,5 à 50 % en volume, sur la base du volume total de l'adhésif.

7. Adhésif électroconducteur selon l'une quelconque des revendications précédentes, dans lequel le monomère (méth)acrylate est présent dans l'adhésif en une quantité dans la plage de 60 à 90 % en poids, de préférence de 65 à 75 % en poids, sur la base du poids total des composés organiques dans l'adhésif, et/ou
dans lequel le polymère est présent dans l'adhésif en une quantité dans la plage de 10 à 30 % en poids, de préférence 12 à 25 % en poids, sur la base du poids total des composés organiques dans l'adhésif, et/ou
dans lequel l'initiateur de polymérisation est présent dans l'adhésif en une quantité dans la plage allant de 0,1 à 3,0 % en poids, de préférence de 0,5 à 2,5 % en poids, sur la base du poids total des composés organiques dans l'adhésif.

8. Adhésif électroconducteur selon l'une quelconque des revendications précédentes, dans lequel l'adhésif comprend un apprêt, de préférence en une quantité dans la plage de 0,5 à 5,0 % en poids, de préférence de 1,5 à 3,5 % en poids, sur la base du poids total des composés organiques dans l'adhésif.

9. Adhésif électroconducteur selon l'une quelconque des revendications précédentes, dans lequel l'adhésif comprend un agent de réticulation, de préférence en une quantité dans la plage de 1,0 à 15 % en poids, de préférence de 3,0 à 10 % en poids, sur la base du poids total des composés organiques dans l'adhésif.

10. Adhésif électroconducteur selon l'une quelconque des revendications précédentes, dans lequel l'adhésif comprend un activateur de polymérisation, de préférence en une quantité de 0,05 à 3,0 % en poids, de préférence de 0,2 à 2,0 % en poids, sur la base du poids total des composés organiques dans l'adhésif.

11. Adhésif électroconducteur selon l'une quelconque des revendications précédentes, dans lequel l'initiateur de polymérisation est un composé ayant une température de décomposition auto-accélérée (TDAC) dans la plage de 35 à 100 °C, de préférence dans la plage de 50 à 85 °C, et/ou dans lequel l'initiateur de polymérisation est un peroxyde, un composé diazo, un barbiturate, ou un photoinitiateur, et de préférence est un peroxyde.

12. Adhésif électroconducteur selon l'une quelconque des revendications précédentes, dans lequel l'adhésif comprend les composants suivants dans les quantités relatives suivantes :
| | | |
|---|---|---|
| monomère (méth)acrylate | | 60 à 100 parties en poids, de préférence 70 à 90 parties en poids, |
| polymère b) | | 10 à 30 parties en poids, de préférence 16 à 24 parties en poids, |
| initiateur | | 1,0 à 3,0 parties en poids, de préférence 1,6 à 2,4 parties en poids, |
| facultativement un agent de réticulation | | 3,0 à 9,0 parties en poids, de préférence 5,0 à 7,0 parties en poids, |
| facultativement un apprêt | | 0,8 à 4,0 parties en poids, de préférence 2,0 à 3,5 parties en poids. |

13. Trousse permettant de préparer un adhésif électroconducteur, de préférence un adhésif électroconducteur selon l'une quelconque des revendications 1 à 12, dans laquelle la trousse comprend un composant A et un composant B, dans laquelle le composant A comprend un monomère (méth)acrylate, et dans laquelle le composant B comprend
un polymère étant soluble dans le monomère (méth)acrylate du composant A, et dans laquelle le polymère est choisi dans le groupe constitué par les poly((méth)acrylates), les copolymères de poly((méth)acrylate), et un mélange de ceux-ci,
un métal biocompatible ayant une taille médiane de particules d₅₀ inférieure à 50 µm, et
un initiateur de polymérisation.

14. Dispositif médical implantable comprenant au moins deux pièces électroniques, dans lequel les au moins deux pièces électroniques sont connectées par
a) l'adhésif électroconducteur selon l'une quelconque des revendications 1 à 12, ou une forme durcie de celui-ci, ou
b) l'adhésif électroconducteur préparé à partir de la trousse selon la revendication 13, ou une forme durcie de celui-ci.

15. Utilisation d'un adhésif électroconducteur selon l'une quelconque des revendications 1 à 12, ou utilisation d'une trousse selon la revendication 13, pour connecter au moins deux pièces électroniques dans un dispositif médical implantable.
